# EUROPÄISCHE PATENTSCHRIFT

(11) **EP 2 994 089 B1**
(45) Veröffentlichungstag und Bekanntmachung des Hinweises auf die Patenterteilung: **15.03.2017**
(21) Anmeldenummer: 14721812.7
(22) Anmeldetag: 29.04.2014
(51) Int. Cl.: A61K 8/04, A61K 8/37, A61Q 15/00

(54) **AEROSOLZUSAMMENSETZUNG MIT VERBESSERTEN SPRÜHEIGENSCHAFTEN**
AEROSOL COMPOSITION HAVING IMPROVED SPRAYING PROPERTIES
COMPOSITION D'AÉROSOL PRÉSENTANT DES PROPRIÉTÉS DE PULVÉRISATION AMÉLIORÉES

(30) Priorität: 07.05.2013 DE 102013208337
(43) Veröffentlichungstag der Anmeldung: 16.03.2016
(73) Patentinhaber: Henkel AG & Co. KGaA, 40589 Düsseldorf (DE)
(72) Erfinder: DÖRING, Thomas, 41540 Dormagen (DE); TECKENBROCK, Gertraud, 45549 Sprockhövel (DE)
(86) Internationale Anmeldenummer: PCT/EP2014/058698
(87) Internationale Veröffentlichungsnummer: WO 2014/180710

(56) Entgegenhaltungen:
- WO-A2-2006/004931
- DE-A1-102009 002 098
- DE-A1-102009 029 669
- DE-A1-102011 083 293
- DE-A1-102011 083 872

## Beschreibung

Die Erfindung betrifft im Wesentlichen wasserfreie Aerosolzusammensetzungen, die mit Hilfe eines Treibmittels versprüht werden können, und die eine spezielle Kombination zweier unterschiedlicher Ester enthalten.

Das Waschen, Reinigen und Pflegen des menschlichen Körpers stellt ein wichtiges Grundbedürfnis dar, und die Kosmetikhersteller versuchen stets, den sich fortwährend verändernden und weiterentwickelnden Bedürfnissen der Verbraucher durch die Bereitstellung neuartiger und/oder verbesserter Produkte gerecht zu werden.
Ein grundlegender Bestandteil der täglichen Hygiene ist beispielsweise die effektive Beseitigung oder zumindest die signifikante Reduzierung des Körper- und/oder Schweißgeruchs. Diese Gerüche entstehen durch die bakterielle Zersetzung verschiedener Bestandteile des menschlichen (apokrinen) Schweißes auf der Haut, bei der u.a. niedere Fettsäuren (insbesondere C₄₋₁₀-Fettsäuren), Ammoniak, Amine, Indole und schwefelhaltige Substanzen entstehen.

Zur Bekämpfung von Körper- und/oder Schweißgeruch werden im Handel Produkte in den verschiedensten Darreichungsformen angeboten, beispielsweise Puder, Stifte, Aerosolsprays, Pumpsprays, flüssige und gelförmige Roll-ons, Cremes, Gele sowie getränkte flexible Substrate (sogenannte Deotücher).

Eine von vielen Verbrauchern besonders bevorzugte Variante von Deodorant- und Antitranspirantzusammensetzungen sind Treibmittel-getriebene Aerosolsprays.
Treibmittel enthaltende Aerosolsprays werden aber auch in anderen Bereichen der Körperpflege gewünscht und benötigt, wie beispielsweise für die Frisurgestaltung und/oder Fixierung von Haaren, für das Auftragen eines UV-Schutzmittels oder einer Tönung auf die Haut oder für das Auftragen von Parfums auf die Haut.
Sie zeichnen sich durch eine bequeme Handhabung und Dosierbarkeit, eine hygienische Anwendung und gleich bleibende Wirksamkeit des gegen die Außenatmosphäre abgeschlossenen Inhalts aus.

Handelsübliche Aerosol-Abgabevorrichtungen umfassen stets eine Aerosoldose und einen Sprühkopf. Sie stehen unter Druck und enthalten hohe Mengen an Treibmitteln.
Diese Anordnung ist notwendig um sicherzustellen, dass die Sprüheigenschaften über die gesamte Gebrauchsdauer des Aerosolsprays erhalten bleiben. Anderenfalls könnte es beispielsweise zu Ventilverstopfungen kommen.

Ein Nachteil der zuvor genannten Anordnung ist jedoch oftmals eine unbefriedigende Fokussierung des Sprühstrahls. Bedingt durch den hohen Druck und eine besonders feine Partikelverteilung kann mitunter ein regelrechter Sprühnebel entstehen, der von den Verbrauchern als unangenehm empfunden wird, da er sowohl unerwünschte Produktverluste, als auch eine überhöhte Aufbringrate des Aerosols zur Folge haben kann.

In Extremfällen konnte durch die Bildung eines Sprühnebels (bzw. einer "Deowolke") sogar beobachtet werden, dass die Schweißreduktion eines Antritranspirant-Aerosols geringer war als die Schweißreduktion eines auf den gleichen Deo- und/oder Antitranspirantwirkstoffen basierenden Stiftes oder Roll-ons, obwohl das Antitranspirant-Aerosol insgesamt eine höhere Wirkstoffkonzentrationen aufwies.

Die Aufgabe der vorliegenden Erfindung bestand folglich darin, Aerosolzusammensetzungen bereitzustellen, die die o.g. Nachteile nicht aufweisen bzw. minimieren.

Insbesondere sollten Treibmittel enthaltende Aerosole bereitgestellt werden, die bei gleich bleibender Sprühmenge eine verbesserte Aufbringrate von Wirkstoffen auf der Anwendungsoberfläche gewährleisten.

Überraschenderweise wurde gefunden, dass die Sprüheigenschaften Treibmittel-getriebener Aerosole besser gesteuert werden können, wenn den Aerosolen eine Kombination mindestens zweier unterschiedlicher Ester in einem bestimmten Mengenverhältnis hinzugefügt wird.

Ein erster Gegenstand der vorliegenden Erfindung ist daher eine Aerosolzusammensetzung für die Körperpflege, die
- mindestens ein Treibmittel und
- mindestens eine Zusammensetzung A, enthaltend
   a) mindestens einen Ester, der aus mindestens einem linearen oder verzweigten, gesättigten oder ungesättigten Alkohol mit 4 bis 30 Kohlenstoffatomen und mindestens einer linearen oder verzweigten, gesättigten oder ungesättigten Carbonsäure mit 8 bis 30 Kohlenstoffatomen gebildet wird, und
   b) mindestens einen Ester, der aus mindestens einer C₂-C₇-Mono-, -Di- oder -Tricarbonsäure, die gegebenenfalls eine oder mehrere Hydroxylgruppen enthalten kann, und mindestens einem linearen oder verzweigten, gesättigten oder ungesättigten Alkohol mit 1 bis 30 Kohlenstoffatomen gebildet wird, enthält,
wobei die Zusammensetzung A maximal 2 Gew.-% freies Wasser, bezogen auf das Gesamtgewicht der Zusammensetzung A, enthält, und wobei das Gewichtsverhältnis der Ester a) : b) in der Zusammensetzung A 3,5 : 1 bis 1,5 : 1 beträgt. Ein zweiter Gegenstand der Erfindung ist eine desodorierende Aerosolzusammensetzung für die Körperpflege, die - bezogen auf ihr Gesamtgewicht -
- 10 bis 90 Gew.-% mindestens eines Treibmittels, ausgewählt aus Propan, n-Butan, Isobutan, n-Pentan, Isopentan, Dimethylether, Kohlendioxid, Distickstoffoxid, Fluorkohlenwasserstoffen und/oder Fluorchlorkohlenwasserstoffen, und
- 1 bis 40 Gew.-% mindestens eine Zusammensetzung A, enthaltend
   a) mindestens einen Ester, der aus mindestens einem linearen oder verzweigten, gesättigten oder ungesättigten Alkohol mit 4 bis 30 Kohlenstoffatomen und mindestens einer linearen oder verzweigten, gesättigten oder ungesättigten Carbonsäure mit 8 bis 30 Kohlenstoffatomen gebildet wird,
   b) mindestens einen Ester, der aus mindestens einer C₂-C₇-Mono-, -Di- oder -Tricarbonsäure, die gegebenenfalls eine oder mehrere Hydroxylgruppen enthalten kann, und mindestens einem linearen oder verzweigten, gesättigten oder ungesättigten Alkohol mit 1 bis 30 Kohlenstoffatomen gebildet wird, und
   c) mindestens einen schweißhemmenden Wirkstoff, enthält,
wobei die Zusammensetzung A maximal 2 Gew.-% freies Wasser, bezogen auf das

Gesamtgewicht der Zusammensetzung A, enthält, und wobei das Gewichtsverhältnis der Ester a) : b) in der Zusammensetzung A 3,5 : 1 bis 1,5 : 1 beträgt.

Geeignete Treibmittel des ersten Erfindungsgegenstandes sind bevorzugt ausgewählt aus Treibgasen der folgenden Gruppe: Propan, n-Butan, Isobutan, n-Pentan, Isopentan, Dimethylether, Kohlendioxid, Distickstoffoxid, Fluorkohlenwasserstoffen und/oder Fluorchlorkohlenwasserstoffen. Besonders bevorzugte Treibgase sind Propan, n-Butan, Isobutan, n-Pentan, Isopentan und/oder deren Gemische, insbesondere bevorzugt ist ein Gemisch aus Propan und n-Butan in einem bevorzugten Gewichtsverhältnis von Propan/n-Butan von 10-40/60-90, besonders bevorzugt von 10-20/80-90.

Das oder die Treibmittel kann (können) in den Aerosolzusammensetzungen des ersten Erfindungsgegenstandes bevorzugt in Mengen von 10 bis 90 Gew.-%, mehr bevorzugt von 20 bis 90 Gew.-%, besonders bevorzugt von 30 bis 90 Gew.-% und insbesondere von 60 bis 90 Gew.-% enthalten sein, wobei sich die Mengen auf die gesamte Aerosolzusammensetzung beziehen.

Desodorierende Aerosolzusammensetzungen des zweiten Erfindungsgegenstandes enthalten bevorzugt Treibgase aus der Gruppe Propan, n-Butan, Isobutan, n-Pentan, Isopentan und/oder deren Gemische, insbesondere bevorzugt Gemische aus Propan und n-Butan in einem bevorzugten Gewichtsverhältnis von Propan/n-Butan von 10-40/60-90, besonders bevorzugt von 10-20/80-90.

Die Treibgase werden in den desodorierenden Aerosolzusammensetzungen des zweiten Erfindungsgegenstandes bevorzugt in Mengen von 20 bis 90 Gew.-%, besonders bevorzugt von 30 bis 90 Gew.-% und insbesondere von 60 bis 90 Gew.-% eingesetzt, wobei sich die Mengen auf die gesamte desodorierende Aerosolzusammensetzung beziehen.

Erfindungsgemäß bevorzugte Ester a) des ersten und zweiten Erfindungsgegenstandes sind ausgewählt aus Estern mindestens eines verzweigten Alkohols mit 4 bis 20, mehr bevorzugt mit 5 bis 18 und insbesondere mit 6 bis 16 Kohlenstoffatomen und mindestens einer linearen oder verzweigten, gesättigten oder ungesättigten Carbonsäure mit 8 bis 24, bevorzugt mit 10 bis 22 und insbesondere bevorzugt mit 12 bis 20 Kohlenstoffatomen.

Beispiele für besonders bevorzugte Ester a) des ersten und zweiten Erfindungsgegenstandes sind 2-Ethylhexyllaurat, 2-Ethylhexylmyristat, 2-Ethylhexylpalmitat, 2-Ethylhexylcocoat, 2-Ethylhexylstearat, 2-Ethylhexylisostearat, Hexyldecyllaurat, Hexyldecylstearat, Isooctylstearat, Isononylisononanoat, Isononylstearat, Isotridecylnonanoat, 2-Octyldodecylpalmitat, Isocetylstearat sowie Mischungen dieser Ester.

Ganz besonders bevorzugte Ester a) des ersten und zweiten Erfindungsgegenstandes sind 2-Ethylhexyllaurat, 2-Ethylhexylmyristat, 2-Ethylhexylpalmitat, 2-Ethylhexylstearat, 2-Ethylhexylisostearat und/oder Hexyldecylstearat.

Unter den zuvor genannten Estern sind diejenigen ganz besonders bevorzugt, die unter Normalbedingungen flüssig sind.

Insbesondere bevorzugt ist 2-Ethylhexylpalmitat.

Unter erfindungsgemäß geeigneten Estern b) des ersten und zweiten Erfindungsgegenstandes sind alle optischen Formen der zuvor genannten Ester zu verstehen.

Erfindungsgemäß bevorzugte Ester b) des ersten und zweiten Erfindungsgegenstandes sind ausgewählt aus mindestens einer C₂-C₇-Mono-, -Di- oder -Tricarbonsäure, die gegebenenfalls eine oder mehrere Hydroxylgruppen enthalten kann, und mindestens einem linearen oder verzweigten, gesättigten oder ungesättigten Alkohol mit 1 bis 10, bevorzugt mit 1 bis 7 und insbesondere mit 1 bis 4 Kohlenstoffatomen.

Mehr bevorzugte Ester b) des ersten und zweiten Erfindungsgegenstandes sind ausgewählt aus den Methyl-, Ethyl, n-Propyl-, Isopropyl-, n-Butyl-, 2-Butyl- oder den tert.-Butylestern der Glycolsäure, Milchsäure, Äpfelsäure, Weinsäure, Zitronensäure, Oxalsäure, Malonsäure, Bernsteinsäure, Glutarsäure und/oder Adipinsäure.

Beispiele für besonders bevorzugte Ester b) des ersten und zweiten Erfindungsgegenstandes sind die Methyl-, Ethyl- und Isopropylester der Milchsäure, Weinsäure, Oxalsäure, Malonsäure, Bernsteinsäure, Adipinsäure und Zitronensäure wie beispielsweise Ethyllactat, Dimethyltartart, Diethyltartrat, Dimethxyloxalat, Diethyloxalat, Dimethylmalonat, Diethylmalonat, Dimethylsuccinat, Diethylsuccinat, Dimethyladipat, Diethyladipat, Diisopropyladipat, Trimethylcitrat und Triethylcitrat. Unter den zuvor genannten Estern sind diejenigen Ester ganz besonders bevorzugt, die unter Normalbedingungen flüssig sind und bevorzugt einen Siedepunkt > 150°C, mehr bevorzugt > 175°C, besonders bevorzugt > 200°C und insbesondere > 225°C aufweisen.

Insbesondere bevorzugt ist Triethylcitrat.

Eine erste bevorzugte Ausführungsform des ersten Erfindungsgegenstandes und zweiten Erfindungsgegenstandes ist dadurch gekennzeichnet, dass
- der Ester a) aus mindestens einem verzweigten Alkohol mit 4 bis 20, bevorzugt mit 5 bis 18 und insbesondere mit 6 bis 16 Kohlenstoffatomen und mindestens einer linearen oder verzweigten, gesättigten oder ungesättigten Carbonsäure mit 8 bis 24, bevorzugt mit 10 bis 22 und insbesondere mit 12 bis 20 Kohlenstoffatomen gebildet wird, und
- der Ester b) aus mindestens einer C₂-C₇-Mono-, -Di- oder -Tricarbonsäure, die gegebenenfalls eine oder mehrere Hydroxylgruppen enthalten kann, und mindestens einem linearen oder verzweigten, gesättigten oder ungesättigten Alkohol mit 1 bis 10, bevorzugt mit 1 bis 7 und insbesondere mit 1 bis 4 Kohlenstoffatomen gebildet wird.

Innerhalb dieser Ausführungsform ist es besonders bevorzugt, wenn
- der Ester a) ausgewählt ist aus 2-Ethylhexyllaurat, 2-Ethylhexylmyristat, 2-Ethylhexylpalmitat, 2-Ethylhexylcocoat, 2-Ethylhexylstearat, 2-Ethylhexylisostearat, Hexyldecyllaurat, Hexyldecylstearat, Isooctylstearat, Isononylisononanoat, Isononylstearat, Isotridecylnonanoat, 2-Octyldodecylpalmitat, Isocetylstearat und/oder Mischungen dieser Ester, und
- der Ester b) ausgewählt ist aus den Methyl-, Ethyl, n-Propyl-, Isopropyl-, n-Butyl-, 2-Butyl- oder den tert.-Butylestern der Glycolsäure, Milchsäure, Äpfelsäure, Weinsäure, Zitronensäure, Oxalsäure, Malonsäure, Bernsteinsäure, Glutarsäure und/oder Adipinsäure.

Innerhalb dieser Ausführungsform ist es ganz besonders bevorzugt, wenn
- der Ester a) ausgewählt ist aus 2-Ethylhexyllaurat, 2-Ethylhexylmyristat, 2-Ethylhexylpalmitat, 2-Ethylhexylstearat, 2-Ethylhexylisostearat und/oder Hexyldecylstearat, und
- der Ester b) ausgewählt ist aus Ethyllactat, Dimethyltartart, Diethyltartrat, Dimethxyloxalat, Diethyloxalat, Dimethylmalonat, Diethylmalonat, Dimethylsuccinat, Diethylsuccinat, Dimethyladipat, Diethyladipat, Diisopropyladipat, Trimethylcitrat und Triethylcitrat,
wobei die Ester a) und b) bevorzugt unter Normalbedingungen flüssig sind.

Innerhalb dieser Ausführungsform ist es insbesondere bevorzugt, wenn der Ester a) 2-Ethylhexylpalmitat und der Ester b) Triethylcitrat enthält.

Eine zweite bevorzugte Ausführungsform des ersten und zweiten Erfindungsgegenstandes ist dadurch gekennzeichnet, dass das Gewichtsverhältnis der Ester a) zu b) in der Zusammensetzung A 3.5 : 1 bis 1,5 : 1 und bevorzugt 3 : 1 bis 2 : 1 beträgt. Innerhalb dieser Ausführungsform sind solche Aerosolzusammensetzungen des ersten und zweiten Erfindungsgegenstandes besonders bevorzugt, die
- als Ester a) 2-Ethylhexyllaurat, 2-Ethylhexylmyristat, 2-Ethylhexylpalmitat, 2-Ethylhexylcocoat, 2-Ethylhexylstearat, 2-Ethylhexylisostearat, Hexyldecyllaurat, Hexyldecylstearat, Isooctylstearat, Isononylisononanoat, Isononylstearat, Isotridecylnonanoat, 2-Octyldodecylpalmitat, Isocetylstearat und/oder Mischungen dieser Ester, und
- als Ester b) einen Methyl-, Ethyl, n-Propyl-, Isopropyl-, n-Butyl-, 2-Butyl- oder tert.-Butylester der Glycolsäure, Milchsäure, Äpfelsäure, Weinsäure, Zitronensäure, Oxalsäure, Malonsäure, Bernsteinsäure, Glutarsäure und/oder Adipinsäure enthalten,
wobei das Gewichtsverhältnis der Ester a) zu b) in der Zusammensetzung A 3,5 : 1 bis 1,5 : 1, besonders bevorzugt 3 : 1 bis 2 : 1 beträgt.

Innerhalb dieser Ausführungsform ist es ganz besonders bevorzugt, wenn die Zusammensetzung A des ersten und zweiten Erfindungsgegenstandes
- als Ester a) 2-Ethylhexyllaurat, 2-Ethylhexylmyristat, 2-Ethylhexylpalmitat, 2-Ethylhexylstearat, 2-Ethylhexylisostearat und/oder Hexyldecylstearat, und
- als Ester b) Ethyllactat, Dimethyltartart, Diethyltartrat, Dimethxyloxalat, Diethyloxalat, Dimethylmalonat, Diethylmalonat, Dimethylsuccinat, Diethylsuccinat, Dimethyladipat, Diethyladipat, Diisopropyladipat,Trimethylcitrat und/oder Triethylcitrat enthält,
wobei das Gewichtsverhältnis der Ester a) zu b) in der Zusammensetzung A bevorzugt 3 : 1 bis 2 : 1, besonders bevorzugt von 2,75 : 1 bis 2,25 : 1 beträgt.

Innerhalb dieser Ausführungsform ist es insbesondere bevorzugt, wenn die Zusammensetzung A des ersten und zweiten Erfindungsgegenstandes 2-Ethylhexylpalmitat (Ester a)) und Triethylcitrat (Ester b)) in einem Gewichtsverhältnis von bevorzugt 3 : 1 bis 2 : 1, besonders bevorzugt von 2,75 : 1 bis 2,25 : 1, enthält.

Erfindungsgemäß weiterhin bevorzugte Aerosolzusammensetzungen des ersten und zweiten Erfindungsgegenstandes enthalten
- den mindestens einen Ester a) bevorzugt in Mengen von 1 bis 30 Gew.-%, mehr bevorzugt von 2 bis 20 Gew.-%, besonders bevorzugt von 3 bis 15 Gew.-% und insbesondere bevorzugt von 5 bis 10 Gew.-%, und
- den mindestens einen Ester b) bevorzugt in Mengen von 1 bis 10 Gew.-%, mehr bevorzugt von 1 bis 7,5 Gew.-%, besonders bevorzugt von 1 bis 5 Gew.-% und besonders bevorzugt von 1 bis 3 Gew.-%,
wobei sich die Mengenangaben auf das Gesamtgewicht der Zusammensetzung A beziehen.

In einer dritten bevorzugten Ausführungsform können Aerosolzusammensetzungen des ersten und zweiten Erfindungsgegenstandes in der Zusammensetzung A zusätzlich mindestens ein weiteres, von a) und b) verschiedenes, unter Normalbedingungen flüssiges Öl als Träger enthalten.

Geeignete Öle der dritten bevorzugten Ausführungsform werden in den Zusammensetzungen A des ersten und zweiten Erfindungsgegenstandes bevorzugt in Mengen von 1 - 80 Gew.-%, mehr bevorzugt von 10 - 75 Gew.-%, besonders bevorzugt von 20 bis 65 Gew.-% und insbesondere bevorzugt von 30 - 60 Gew.-%, jeweils bezogen auf das Gesamtgewicht der Zusammensetzung A, eingesetzt.

Besonders geeignete Öle der dritten bevorzugten Ausführungsform bestehen zu mindestens 90 Gew.-% aus bei 20 °C flüssigen Ölkomponenten.

Besonders geeignete Öle der dritten bevorzugten Ausführungsform sind ausgewählt aus:
- flüchtigen Siliconölen, die cyclisch sein können, wie z. B. Octamethylcyclotetrasiloxan, Decamethylcyclopentasiloxan und Dodecamethylcyclohexasiloxan sowie Mischungen hiervon, wie sie z. B. in den Handelsprodukten DC 244, 245, 344 und 345 von Dow Corning enthalten sind, oder linear, z. B. Hexamethyldisiloxan (L₂), Octamethyltrisiloxan (L₃), Decamethyltetrasiloxan (L₄), beliebige Zweier- und Dreiermischungen aus L₂, L₃ und/oder L₄, wie sie z. B. in den Handelsprodukten DC 2-1184, Dow Corning^{®} 200 (0, 65 cSt) und Dow Corning^{®} 200 (1,5 cSt) von Dow Corning enthalten sind,
- nichtflüchtigen höhermolekularen linearen Dimethylpolysiloxanen, im Handel erhältlich z. B. unter der Bezeichnung Dow Corning^{®} 190, Dow Corning^{®} 200 Fluid mit Viskositäten im Bereich von 5-100 cSt, bevorzugt 5-50 cSt oder auch 5-10 cSt, und Baysilon^{®} 350 M;
- den Estern eines linearen oder verzweigten, gesättigten oder ungesättigten Alkohols mit 2 - 5 Kohlenstoffatomen mit einer linearen oder verzweigten, gesättigten oder ungesättigten Carbonsäure mit 10 - 30 Kohlenstoffatomen, wie beispielsweise Isopropylmyristat, Isopropylpalmitat, Isopropylstearat, Isopropylisostearat, Isopropyloleat, n-Butylstearat, Ethylenglycoldioleat und -dipalmitat;
- den Benzoesäureestern von linearen oder verzweigten C₈-₂₂-Alkanolen, z. B. die Handelsprodukte Finsolv^{®} TN (C₁₂-C₁₅-Alkylbenzoat), Finsolv^{®} SB (Isostearylbenzoat) und Finsolv^{®} EB (Ethylhexylbenzoat);
- den Anlagerungsprodukten von Ethylenoxid und/oder Propylenoxid an ein- oder mehrwertige C₃₋₂₀-Alkanole wie Butanol, Butandiol, Myristylalkohol und Stearylalkohol, z. B. PPG-14-Butylether (Ucon Fluid^{®} AP), PPG-9-Butylether (Breox^{®} B25), PPG-10-Butandiol (Macol^{®} 57), PPG-3-Myristylether (Witconol^{®} APM) und PPG-15-Stearylether (Arlamol^{®} E);
- flüssigen Paraffinölen, Isoparaffinölen, z. B. die Handelsprodukte der Permethyl^{®}-Serie, insbesondere Isododecan, Isohexadecan und Isoeicosan, und synthetischen Kohlenwasserstoffen wie Polyisobuten oder Polydecene und alicyclischen Kohlenwasserstoffen, z. B. das Handelsprodukt 1,3-Di-(2-ethyl-hexyl)-cyclohexan (Cetiol^{®} S);
- den verzweigten gesättigten oder ungesättigten Fettalkoholen mit 6-30 Kohlenstoffatomen. Diese Alkohole werden häufig auch als Guerbet-Alkohole bezeichnet, da sie nach der Guerbet-Reaktion erhältlich sind. Besonders bevorzugte Alkoholöle sind beispielsweise Hexyldecanol (Eutanol^{®} G), Octyldodecanol und 2-Ethylhexylalkohol;
- Mischungen aus Guerbetalkoholen und Guerbetalkoholestern, z. B. das Handelsprodukt Cetiol^{®} PGL (Hexyldecanol und Hexyldecyllaurat).
- den symmetrischen, unsymmetrischen oder cyclischen Estern der Kohlensäure mit Fettalkoholen, beispielsweise Glycerincarbonat, Dicaprylylcarbonat (Cetiol^{®} CC) oder die Ester der DE-OS 197 56 454;
- Triglyceriden von linearen oder verzweigten, gesättigten oder ungesättigten, gegebenenfalls hydroxylierten C₈₋₃₀-Fettsäuren. Besonders geeignet kann die Verwendung natürlicher Öle, z.B. Sojaöl, Baumwollsaatöl, Sonnenblumenöl, Palmöl, Palmkernöl, Leinöl, Mandelöl, Rizinusöl, Maisöl, Olivenöl, Rapsöl, Sesamöl, Distelöl, Weizenkeimöl, Pfirsichkernöl und die flüssigen Anteile des Kokosöls und dergleichen sein. Geeignet sind aber auch synthetische Triglyceridöle, insbesondere Capric/Caprylic Triglycerides, z. B. die Handelsprodukte Myritol^{®} 318, Myritol^{®} 331 (Cognis) oder Miglyol^{®} 812 (Hüls) mit unverzweigten Fettsäureresten sowie Glyceryltrüsostearin und die Handelsprodukte Estol^{®} GTEH 3609 (Uniqema) oder Myritol^{®} GTEH (Cognis) mit verzweigten Fettsäureresten;
- Dicarbonsäureestern von linearen oder verzweigten C₂-C₁₀-Alkanolen, insbesondere Diisopropyladipat, Di-n-butyladipat, Di-(2-ethylhexyl)adipat, Dioctyladipat, Diethyl-/Di-n-butyl/ Dioctylsebacat, Diisopropylsebacat, Dioctylmalat, Dioctylmaleat, Dicaprylylmaleat, Diisooctylsuccinat, Di-2-ethylhexylsuccinat und Di-(2-hexyldecyl)-succinat;
- Di-n-alkylethern mit insgesamt 12 bis 36, insbesondere 12 bis 24 C-Atomen, z. B. Di-n-octylether (Cetiol^{®} OE), Di-n- n-Hexyl-n-octylether und n-Octyl-n-decylether.

Ganz besonders bevorzugte Öle der dritten bevorzugten Ausführungsform sind die flüchtigen cyclischen Siliconöle Decamethylcyclopentasiloxan und Dodecamethylcyclohexasiloxan, die flüchtigen linearen Siliconöle Hexamethyldisiloxan (L₂), Octamethyltrisiloxan (L₃) und Decamethyltetrasiloxan (L₄) sowie beliebige Zweier- und Dreiermischungen aus L₂, L₃ und/oder L₄, flüchtige und nichtflüchtige lineare Siliconöle aus der Serie Dow Corning 200 Fluid mit Viskositäten von 0,65, 1,0, 1,5 und 5 cSt, die Esteröle Isopropylmyristat und Isopropylpalmitat, die Benzoesäureester von linearen oder verzweigten C₈-₂₂-Alkanolen, insbesondere das Handelsprodukt Finsolv^{®} TN (C₁₂-C₁₅-Alkylbenzoat), C₁₂-C₁₅-Alkyllactat, Di-C₁₂-C₁₃-Alkylmalat, PPG-14-Butylether (Ucon Fluid^{®} AP), die Handelsprodukte der Permethyl^{®}-Serie, insbesondere Isododecan, Isohexadecan und Isoeicosan, sowie Polyisobuten und Polydecene sowie Mischungen der genannten Komponenten.

Insbesondere bevorzugte Öle der dritten bevorzugten Ausführungsform sind flüchtige cyclische Silikonöle wie Decamethylcyclopentasiloxan und Dodecamethylcyclohexasiloxan, insbesondere Decamethylcyclopentasiloxan, Esteröle wie Isopropylmyristat und Isopropylpalmitat, insbesondere Isopropylmyristat, und Benzoesäureester von linearen oder verzweigten C₈-₂₂-Alkanolen, insbesondere das Handelsprodukt Finsolv^{®} TN (C₁₂-C₁₅-Alkylbenzoat).

Es kann erfindungsgemäß bevorzugt sein, Mischungen der vorgenannten Öle einzusetzen. Dabei sind besonders Mischungen aus zwei Ölkomponententypen, z. B. ein flüchtiges Siliconöl und Esteröl, bevorzugt. Besonders bevorzugt sind Ölmischungen, die mindestens ein flüchtiges cyclisches Siliconöl enthalten. Außerordentlich bevorzugt sind Ölmischungen, die überwiegend, das heißt zu mehr als 50 Gew.-%, mindestens eines flüchtigen cyclischen Siliconöls enthalten. Weiterhin bevorzugt sind Ölmischungen, die 50 - 95 Gew.-%, besonders bevorzugt 60 - 90 Gew.-%, mindestens eines flüchtigen cyclischen Siliconöls in Kombination mit 5 - 40 Gew.-%, besonders bevorzugt 10 - 30 Gew.-%, mindestens eines Esteröls, insbesondere Isopropylmyristat und/oder Isopropylpalmitat, enthalten, wobei sich die Mengenangaben auf das Gesamtgewicht der Zusammensetzung A beziehen.

Besonders bevorzugte Aerosolzusammensetzungen der dritten bevorzugten Ausführungsform enthalten eine Mischung aus mindestens zwei von a) und b) verschiedenen, unter Normalbedingungen flüssigen Ölen, insbesondere bevorzugt eine Mischung aus mindestens zwei der folgenden Öle: flüchtige cyclische Silikonöle wie Decamethylcyclopentasiloxan und/oder Dodecamethylcyclohexasiloxan, Esteröle wie Isopropylmyristat und/oder Isopropylpalmitat, und/oder Benzoesäureester von linearen oder verzweigten C₈-₂₂-Alkanolen.

Insbesondere bevorzugte Aerosolzusammensetzungen der dritten bevorzugten Ausführungsform enthalten eine Mischung aus mindestens zwei von a) und b) verschiedenen, unter Normalbedingungen flüssigen Ölen, bevorzugt eine Mischung, die 50 - 95 Gew.-%, besonders bevorzugt 60 - 90 Gew.-% mindestens eines flüchtigen cyclischen Siliconöls, insbesondere Decamethylcyclopentasiloxan, in Kombination mit 5 - 40 Gew.-%, besonders bevorzugt 10 - 30 Gew.-% mindestens eines Esteröls, insbesondere Isopropylmyristat, enthält, wobei sich die Mengenangaben auf das Gesamtgewicht der Zusammensetzung A beziehen.

Desodorierende Aerosolzusammensetzungen des zweiten Erfindungsgegenstandes sind dadurch gekennzeichnet, dass sie neben einem Treibmittel und der Esterkombination a) und b) mindestens einen schweißhemmenden (Antritranspirant-)Wirkstoff enthalten.

Erfindungsgemäß bevorzugte schweißhemmende (Antitranspirant-)Wirkstoffe sind die wasserlöslichen adstringierenden anorganischen und organischen Salze des Aluminiums, Zirkoniums und Zinks bzw. beliebige Mischungen dieser Salze. Besonders bevorzugte Antitranspirant-Wirkstoffe sind ausgewählt aus den Aluminiumchlorhydraten, zum Beispiel Aluminiumsesquichlorhydrat, Aluminiumchlorhydrex-Propylenglykol (PG) oder -Polyethylenglykol (PEG), Aluminiumsesquichlorhydrex-PG oder -PEG, Aluminium-PG-dichlorhydrex oder Aluminium-PEG-dichlorhydrex, Aluminiumhydroxid, weiterhin ausgewählt aus den Aluminiumzirconiumchlorhydraten, wie Aluminiumzirconiumtrichlorhydrat, Aluminiumzirconiumtetrachlorhydrat, Aluminiumzirconiumpentachlorhydrat, Aluminiumzirconiumoctachlorhydrat, den AluminiumZirkonium-Chlorohydrat-Glycin-Komplexen wie Aluminiumzirconiumtrichlorhydrexglycin, Aluminiumzirconiumtetrachlorhydrexglycin, Aluminiumzirconiumpentachlorhydrexglycin, Aluminiumzirconiumoctachlorhydrexglycin, Kaliumaluminiumsulfat (KAl(SO₄)₂ - 12 H₂O, Alaun), Aluminiumundecylenoylkollagenaminosäure, Natriumaluminiumlactat + Aluminiumsulfat, Natriumaluminiumchlorhydroxylactat, Aluminiumbromhydrat, Aluminiumchlorid, den Komplexen von Zink- und Natriumsalzen, den Komplexen von Lanthan und Cer, den Aluminiumsalzen von Lipoaminosäuren, Aluminiumsulfat, Aluminiumlactat, Aluminiumchlorhydroxyallantoinat, Natrium-Aluminium-Chlorhydroxylactat, Zinkchlorid, Zinksulfocarbolat, Zinksulfat und Zirkoniumchlorohydrat.
Unter "Wasserlöslichkeit" wird erfindungsgemäß eine Löslichkeit von wenigstens 5 Gew.-% in Wasser bei 20 °C verstanden, das heißt, dass Mengen von wenigstens 5 g des Antitranspirant-Wirkstoffes in 95 g Wasser bei 20 °C löslich sind. Die Antitranspirant-Wirkstoffe können als wässrige Lösungen eingesetzt werden.

Besonders bevorzugte erfindungsgemäße desodorierende Aerosolzusammensetzungen sind dadurch gekennzeichnet, dass sie als schweißhemmenden Wirkstoff mindestens einen schweißhemmenden Antitranspirant-Wirkstoff, bevorzugt ausgewählt aus adstringierenden Aluminiumsalzen, insbesondere Aluminiumchlorohydrat, Aluminiumsequichlorohydrat und/oder Aluminiumchlorid, in einer Gesamtmenge von 5 - 60 Gew.-%, vorzugsweise von 10 - 50 Gew.-% und insbesondere 15 - 40 Gew.-%, enthalten, wobei sich die Mengenangaben auf das Gesamtgewicht der Zusammensetzung A beziehen.

In einer vierten bevorzugten Ausführungsform enthält eine desodorierende Aerosolzusammensetzung des zweiten Erfindungsgegenstandes, bezogen auf ihr Gesamtgewicht, daher
(i) 20 bis 90 Gew.-%, besonders bevorzugt von 30 bis 90 Gew.-% und insbesondere von 60 bis 90 Gew.-% mindestens eines Treibgases aus der Gruppe Propan, n-Butan, Isobutan, n-Pentan, Isopentan und/oder deren Gemische,
(ii) 1 bis 40 Gew.-% mindestens eine Zusammensetzung A, enthaltend
   - einen Ester a), ausgewählt aus 2-Ethylhexyllaurat, 2-Ethylhexylmyristat, 2-Ethylhexylpalmitat, 2-Ethylhexylcocoat, 2-Ethylhexylstearat, 2-Ethylhexylisostearat, Hexyldecyllaurat, Hexyldecylstearat, Isooctylstearat, Isononylisononanoat, Isononylstearat, Isotridecylnonanoat, 2-Octyldodecylpalmitat, Isocetylstearat und/oder Mischungen dieser Ester,
   - einen Ester b) ausgewählt aus Methyl-, Ethyl, n-Propyl-, Isopropyl-, n-Butyl-, 2-Butyl- oder tert.-Butylestern der Glycolsäure, Milchsäure, Äpfelsäure, Weinsäure, Zitronensäure, Oxalsäure, Malonsäure, Bernsteinsäure, Glutarsäure und/oder Adipinsäure, und
   - mindestens einen schweißhemmenden Wirkstoff, ausgewählt aus adstringierenden Aluminiumsalzen, insbesondere Aluminiumchlorohydrat, Aluminiumsequichlorohydrat und/oder Aluminiumchlorid,
wobei die Zusammensetzung A maximal 2 Gew.-%, bevorzugt maximal 1 Gew.-% freies Wasser, bezogen auf das Gesamtgewicht der Zusammensetzung A, enthält, und wobei das Gewichtsverhältnis der Ester a) : b) in der Zusammensetzung A 3,5 : 1 bis 1,5 : 1, besonders bevorzugt 3 : 1 bis 2 : 1 beträgt.

In einer weiteren bevorzugten Ausführungsform können die desodorierenden Aerosolzusammensetzungen des zweiten Erfindungsgegenstandes weiterhin mindestens einen desodorierenden Wirkstoff enthalten, der ausgewählt ist aus Geruchsabsorbern, desodorierend wirkenden Ionenaustauschern, keimhemmenden Wirkstoffen, präbiotisch wirksamen Komponenten sowie Inhibitoren der für die Schweißzersetzung verantwortlichen Enzyme oder, besonders bevorzugt, Kombinationen dieser Wirkstoffe.

Silicate können als bevorzugte Geruchsabsorber dienen, die auch gleichzeitig die rheologischen Eigenschaften der erfindungsgemäßen Zusammensetzung A vorteilhaft unterstützen. Zu den besonders bevorzugten Silicaten zählen vor allem Schichtsilicate, und unter diesen insbesondere Montmorillonit, Kaolinit, Ilit, Beidellit, Nontronit, Saponit, Hectorit, Bentonit, Smectit und Talkum. Weitere besonders bevorzugte Geruchsabsorber sind beispielsweise Zeolithe, Zinkricinoleat, Cyclodextrine, bestimmte Metalloxide, wie z. B. Aluminiumoxid, sowie Chlorophyll.
Geruchsabsorber werden in den desodorierenden Aerosolzubereitungen bevorzugt in einer Menge von 0,1 - 10 Gew.-%, besonders bevorzugt von 0,5 - 7 Gew.-% und insbesondere bevorzugt von 1 - 5 Gew.-%, jeweils bezogen auf das Gesamtgewicht der Zusammensetzung A, eingesetzt.

Bevorzugte desodorierende Aerosolzusammensetzungen sind dadurch gekennzeichnet, dass sie in der Zusammensetzung A weiterhin mindestens einen Geruchsabsorber, bevorzugt ein Silicat, enthalten.

Unter keimhemmenden oder antimikrobiellen Wirkstoffen werden solche Wirkstoffe verstanden, die die Zahl der an der Geruchsbildung beteiligten Hautkeime reduzieren bzw. deren Wachstum hemmen. Zu diesen Keimen zählen unter anderem verschiedene Spezies aus der Gruppe der Staphylokokken (z. B. Staphylococcus hominis), der Gruppe der Corynebakterien (z. B. Corynebacterium xerosis, Corynebacterium CDCG2), Anaerokokken (z. B. Anaerococcus octavius) und Mikrokokken.

Als keimhemmende oder antimikrobielle Wirkstoffe können bevorzugt die Riechstoffgemische Protectate HR und Protectate MOD 2 der Firma Symrise dienen.

Das Riechstoffgemisch Protectate HR der Firma Symrise enthält 25 - 50 Gew.-% Phenoxyethanol, 5-10 Gew.-% 2-Methyl-5-phenylpentan-1-ol mit dem Trivialnamen Rosaphen, 34 - 70 Gew.-% 2-Benzylheptan-1-ol mit dem Trivialnamen Jasmol, 1 - 5 Gew.-% 4-Methoxybenzylalkohol (Anisalkohol) und 0,01 - 1 Gew.-% 5-Methyl-2-isopropylphenol (Thymol). Das Riechstoffgemisch Protectate MOD 2 der Firma Symrise enthält 25 - 45 Gew.-% Phenoxyethanol, 5 - 10 Gew.-% 2-Methyl-5-phenylpentan-1-ol und 45 - 70 Gew.-% 2-Benzyl-heptan-1-ol.

Weiterhin sind Organohalogenverbindungen sowie -halogenide, quartäre Ammoniumverbindungen, eine Reihe von Pflanzenextrakten und Zinkverbindungen bevorzugte keimhemmende oder antimikrobielle Wirkstoffe. Hierzu zählen u. a. Triclosan, Chlorhexidin und Chlorhexidingluconat, 3,4,4'-Trichlorcarbanilid, Bromchlorophen, Dichlorophen, Chlorothymol, Chloroxylenol, Hexachlorophen, Dichloro-m-xylenol, Dequaliniumchlorid, Domiphenbromid, Ammoniumphenolsulfonat, Benzalkoniumhalogenide, Benzalkoniumcetylphosphat, Benzalkoniumsaccharinate, Benzethoniumchlorid, Cetylpyridiniumchlorid, Laurylpyridiniumchlorid, Laurylisoquinoliniumbromid, Methylbenzedoniumchlorid. Weiterhin sind Phenol, Phenoxyethanol, Dinatriumdihydroxyethylsulfosuccinylundecylenat, Natriumbicarbonat, Zinklactat, Natriumphenolsulfonat und Zinkphenolsulfonat, Ketoglutarsäure, Terpenalkohole wie z. B. das besonders bevorzugte Farnesol, Chlorophyllin-Kupfer-Komplexe, α-Monoalkylglycerinether mit einem verzweigten oder linearen gesättigten oder ungesättigten, gegebenenfalls hydroxylierten C₆ - C₂₂-Alkylrest, besonders bevorzugt α-(2-Ethylhexyl)glycerinether, im Handel erhältlich als Sensiva^{®} SC 50 (ex Schülke & Mayr), Carbonsäureester des Mono-, Di- und Triglycerins (z. B. Glycerinmonolaurat, Diglycerinmonocaprinat), Lantibiotika sowie Pflanzenextrakte (z. B. grüner Tee und Bestandteile des Lindenblütenöls) bevorzugte desodorierende Wirkstoffe.

Weitere bevorzugte desodorierende Wirkstoffe sind ausgewählt aus sogenannten präbiotisch wirksamen Komponenten, worunter solche Komponenten zu verstehen sind, die nur oder zumindest überwiegend die geruchsbildenden Keime der Hautmikroflora hemmen, nicht aber die erwünschten, das heißt, die nicht-geruchsbildenden Keime, die zu einer gesunden Hautmikroflora gehören. Explizit sind hier die Wirkstoffe zu nennen, die in den Offenlegungsschriften DE 10333245 und DE 10 2004 011 968 als präbiotisch wirksam offenbart sind, dazu gehören Nadelbaumextrakte, insbesondere aus der Gruppe der Pinaceae, und Pflanzenextrakte aus der Gruppe der Sapindaceae, Araliaceae, Lamiaceae und Saxifragaceae, insbesondere Extrakte aus *Picea spp., Paullinia sp., Panax sp., Lamium album* oder *Ribes nigrum* sowie Mischungen dieser Substanzen.

Weitere bevorzugte desodorierende Wirkstoffe sind ausgewählt aus den keimhemmend wirkenden Parfümölen und den Deosafe-Parfümölen, die von der Firma Symrise, vormals Haarmann und Reimer, erhältlich sind.

Desodorierend wirkende Enzyminhibitoren sind solche Stoffe, die die für die Schweißzersetzung verantwortlichen Enzyme, insbesondere die Arylsulfatase, β-Glucuronidase, Aminoacylase, esterspaltende Lipasen und Lipoxigenase, hemmen, wobei Zinkglycinat bevorzugt ist.

Der oder die zuvor genannten desodorierenden Wirkstoff(e) können in den erfindungsgemäßen desodorierenden Aerosolzusammensetzungen bevorzugt in einer Gesamtmenge von 0,1 - 10 Gew.-%, mehr bevorzugt von 0,2 - 7,5 Gew.-%, besonders bevorzugt von 0,3 - 5 Gew.-% und insbesondere bevorzugt von 0,5 - 3,0 Gew.-%, bezogen auf das Gesamtgewicht der Zusammensetzung A, enthalten sein.

Weiterhin bevorzugte erfindungsgemäße desodorierende Aerosolzusammensetzungen sind dadurch gekennzeichnet, dass sie mindestens einen verkapselten und/oder mindestens einen nicht-verkapselten Duftstoff enthalten.

Die Verkapselung der Duftstoffe kann bevorzugt so gewählt sein, dass sie mindestens ein wasserlösliches Verkapselungsmaterial umfasst. Unter Feuchtigkeitseinfluss, hier insbesondere unter dem Einfluss der Hautfeuchtigkeit beziehungsweise des Schweißes, öffnet sich eine gewisse Zeit nach der Applikation das wasserlösliche Verkapselungsmaterial, und der verkapselte Duftstoff sowie gegebenenfalls weitere verkapselte Wirkstoffe, beispielsweise hautkühlende Wirkstoffe, werden nach der Applikation zeitverzögert freigesetzt.
Verkapselte und nicht-verkapselte Duftstoffe, beispielsweise Parfumöle bzw. Parfumöl-Mischungen können gleich oder verschieden sein. Besonders bevorzugte erfindungsgemäße desodorierende Aerosolzusammensetzungen sind dadurch gekennzeichnet, dass sie mindestens einen verkapselten und mindestens einen nicht-verkapselten Duftstoff enthalten, die voneinander verschieden sind.
Bevorzugte erfindungsgemäße desodorierende Aerosolzusammensetzungen sind dadurch gekennzeichnet, dass sie mindestens einen nicht-verkapseltem Duftstoff in einer Gesamtmenge von 0,1 - 3 Gew.%, bevorzugt 0,2 - 1,5 Gew.% und besonders bevorzugt 0,4 - 1 Gew.%, jeweils bezogen auf das Gesamtgewicht der Aerosolzusammensetzung, enthalten.
Weitere bevorzugte erfindungsgemäße desodorierende Aerosolzusammensetzungen sind dadurch gekennzeichnet, dass sie mindestens einen verkapselten Duftstoff in einer Gesamtmenge von 0,01 - 2 Gew.%, bevorzugt 0,1 - 1,0 Gew.% und besonders bevorzugt 0,25 - 0,5 Gew.%, jeweils bezogen auf das Gesamtgewicht der Aerosolzusammensetzung, enthalten.
Als Duftstoffe oder Parfümöle sind Riechstoffverbindungen besonders bevorzugt, z. B. die synthetischen Produkte vom Typ der Ester, Ether, Aldehyde, Ketone, Alkohole und Kohlenwasserstoffe verwendet werden. Zu den bevorzugten phenolischen Riechstoffverbindungen zählt z. B. Carvacrol. Bevorzugte Riechstoffverbindungen vom Typ der Ester sind z. B. Benzylacetat, Methylanthranilat, ortho-t-Butylcyclohexylacetat, p-tert.-Butylcyclohexylacetat, Diethylphthalat, Nonandiol-1,3-diacetat, iso-Nonylacetat, iso-Nonylformiat, Phenylethylphenylacetat, Phenoxyethylisobutyrat, Linalylacetat, Dimethylbenzylcarbinylacetat, Phenylethylacetat, Linalylbenzoat, Benzylformiat, Ethylmethylphenylglycinat, Allylcyclohexylpropionat, Styrallylpropionat, Benzylsalicylat, Ethylsalicylat, iso-Amylsalicylat, Hexylsalicylat und 4-Nonanolid. Zu den bevorzugten Ethern zählen beispielsweise Benzylethylether, zu den bevorzugten Aldehyden z. B. die linearen Alkanale mit 8 bis 18 C-Atomen, Citral, Citronellal, Citronellyloxyacetaldehyd, Cyclamenaldehyd, Hydroxycitronellal, Lilial und Bourgeonal, zu den bevorzugten Ketonen z. B. 6-Acetyl-1,1,3,4,4,6-hexamethyltetrahydronaphthalin, para-t-Amylcyclohexanon, 2-n-Heptylcyclopentanon, β-Methylnaphthylketon und die lonone α-Isomethylionon und Methylcedrylketon, zu den bevorzugten Alkoholen Zimtalkohol, Anethol, Citronellol, Dimyrcetol, Eugenol, Geraniol, Linalool, Phenylethylalkohol und Terpineol, zu den bevorzugten Kohlenwasserstoffen gehören 1,3,4,6,7,8-Hexahydro-4,6,6,7,8,8-hexamethylcyclopenta-a-2-benzopyran, Hydroxymethylisopropylcyclopentan, 3-a-Methyldodecahydro-6,6,9a-trimethylnaphtho-2(2,1-b)furan, iso-Butylchinolin sowie die Terpene und Balsame. Besonders bevorzugt werden Mischungen verschiedener Riechstoffe verwendet, die gemeinsam eine ansprechende Duftnote erzeugen.
Besonders bevorzugte Parfümöle können auch natürliche Riechstoffgemische enthalten, wie sie aus pflanzlichen oder tierischen Quellen zugänglich sind, z.B. Pinien-, Citrus-, Jasmin-, Ylang-Ylang-, Rosen-, oder Lilienöl. Auch ätherische Öle geringerer Flüchtigkeit, die meist als Aromakomponenten verwendet werden, sind als Parfümöle besonders bevorzugt, z. B. Salbeiöl, Kamillenöl, Melissenöl, Minzenöl, Zimtblätteröl, Lindenblütenöl, Wacholderbeerenöl, Vetiveröl, Olibanöl, Galbanumöl, Laudanumöl, Gewürznelkenöl, iso-Eugenol, Thymianöl, Rosenöl, Bergamotteöl und Geraniumöl.

Als Kapselmaterial bevorzugt sind wasserlösliche Polymere wie Stärke, physikalisch und/oder chemisch modifizierte Stärken, Cellulosederivate, wie z. B. Carboxymethylcellulose, Methylcellulose, Hydroxyethylcellulose oder Hydroxypropylmethylcellulose, Carragheene, Alginate, Maltodextrine, Dextrine, Pflanzengummen, Pektine, Xanthane, Polyvinylacetat und Polyvinylalkohol, Polyvinylpyrrolidin, Polyamide, Polyester und Homo- und Copolymere aus Monomeren, ausgewählt aus Acrylsäure, Methacrylsäure, Maleinsäure, Fumarsäure, Itaconsäure sowie den Estern und den Salzen dieser Säuren, sowie beliebige Mischungen dieser Polymeren. Besonders bevorzugte Kapselmaterialien sind chemisch modifizierte Stärken, insbesondere Aluminiumstärkeoctenylsuccinat, z. B. das Handelsprodukt Dry Flo Plus von National Starch, oder Natriumstärkeoctenylsuccinat, z. B. das Handelsprodukt Tylose H 10 von Clariant, desweiteren die Carboxymethylcellulose, Carboxymethylcellulose, Methylcellulose, Hydroxyethylcellulose und Hydroxypropylmethylcellulose, weiterhin Carragheene, Alginate und Maltodextrine, sowie beliebige Mischungen dieser Polymere.
Besonders bevorzugte Kapselmaterialien sind Polymermischungen, die aus chemisch modifizierten Stärken und/oder Hydroxyethylcellulose und einem Anteil von 0,2 - 2 Gew.% an Alginaten und/oder Carragheenen bestehen.

Die Verkapselung kann nach bekannten Verfahren erfolgen. Entsprechende Verfahren sind z. B. offenbart in K. Master, "Spray Drying Handbook", 3. Auflage, John Wiley, 1979. In einem besonders bevorzugten Verkapselungsverfahren wird eine Mischung auf Wasserbasis hergestellt, die etwa 20 - 50 Gew.% des polymeren Verkapselungsmaterials, etwa 0,1 - 2,0 Gew.% eines Emulgators, etwa 5 - 20 Gew.% des zu verkapselten Parfumöls und/oder des zu verkapselten hautkühlenden Wirkstoffs sowie etwa 40 - 60 Gew.% Wasser enthält. Diese Mischung wird homogenisiert und anschließend sprühgetrocknet. Die wirkstoffbeladenen Kapseln werden so als feines Pulver mit einem Teilchendurchmesser von 1 - 150 µm, bevorzugt 20 - 80 µm, besonders bevorzugt 5 - 50 µm, erhalten.
In einem anderen Herstellverfahren erfolgt die Mikroverkapselung durch Koazervation, wobei der Träger bevorzugt aus Gelatine gebildet wird.
Das Kapselmaterial, bestehend aus wasserlöslichen Polymeren und einem geringen Gehalt an Emulgatoren, ermöglicht eine reversible "Wiederverkapselung" der verkapselten Parfumöle und hautkühlenden Wirkstoffe. Die Wiederverkapselung tritt dabei in situ während des Trocknens der Haut, das einer Perspirationsperiode folgt, auf. So treten verschiedene, aufeinander folgende Aktivierungen auf der Haut ein, ohne dass der Benutzer eine weitere Anwendung des erfindungsgemäßen Mittels vornehmen muss.

Erfindungsgemäß können auch Duftstoff- oder Parfüm-freie desodorierende Aerosolzusammensetzungen bevorzugt sein.

Besonders bevorzugte erfindungsgemäße Aerosolzusammensetzungen des ersten und zweiten Erfindungsgegenstandes sind dadurch gekennzeichnet, dass sie im wesentlichen wasserfrei sind, d. h., dass sie maximal 2 Gew.% Wasser, bevorzugt maximal 1 Gew.-% und insbesondere maximal 0,5 Gew.-% freies Wasser enthalten, wobei sich die Mengenangaben auf die Zusammensetzung A, beziehen.

Weiterhin bevorzugte Aerosolzusammensetzungen des ersten und zweiten Erfindungsgegenstandes sind dadurch gekennzeichnet, dass mindestens ein Suspensions- oder Verdickungsmittel, bevorzugt ausgewählt aus hydrophobierten Tonmineralien und pyrogenen Kieselsäuren, enthalten. Bevorzugte hydrophobierte Tonmineralien sind Montmorillonite, Hectorite und Bentonite, insbesondere Disteardimonium Hectorite und Quaternium-18 Hectorite. Die handelsüblichen Verdickungsmittel stellen diese hydrophobierten Tonmineralien in Form eines Gels in Cyclomethicone und gewünschtenfalls einer zusätzlichen Ölkomponente, wie z. B. Propylencarbonat, bereit. Weitere bevorzugte Verdickungsmittel sind pyrogene Kieselsäuren, z. B. die Handelsprodukte der Aerosil®-Serie von Degussa.

Die erfindungsgemäßen Aerosolzusammensetzungen sind bevorzugt in handelsüblichen Aerosoldosen verpackt. Die Dosen können aus Weißblech oder aus Aluminium sein. Weiterhin können die Dosen gemäß einer besonders bevorzugten Ausführungsform innen beschichtet sein, um die Gefahr der Korrosion so gering wie möglich zu halten.
Die Aerosoldosen sind bevorzugt mit einem geeigneten Sprühkopf ausgestattet. Je nach Sprühkopf sind Ausstoßraten, bezogen auf voll gefüllte Dosen, von 0,1 g/s bis 2,0 g/s bevorzugt.

Die Aerosolzusammensetzungen des ersten und zweiten Erfindungsgegenstandes weisen gegenüber handelsüblichen Aerosolzusammensetzungen den Vorteil auf, dass ihr Sprühstrahl besser fokussiert werden kann. Dadurch kann die Aufbringrate und demzufolge auch die Wirksamkeit des jeweiligen Produktes gesteigert werden, denn eine bessere Fokussierung des Sprühstrahls bedingt weniger Sprühnebel.
Ein weiterer Vorteil besteht darin, dass die erfindungsgemäßen Aerosole weniger (durch Sprühwolken verursachte) Produktverluste aufweisen.

Die nachfolgenden Beispiele sollen die Erfindung verdeutlichen, ohne sie hierauf zu beschränken.

### Beispiele:

### 1) Ausführungsbeispiel

Es wurde ein Aerosol-Konzentrat der folgenden Zusammensetzung (vgl. Tabelle 1) hergestellt. Dieses wurde mit einem Propan/n-Butan-Gemisch (15/85) in einem Gewichtsverhältnis von 15 : 85 (Konzentrat: Treibgas) begast und in eine handelsübliche Aerosoldose abgefüllt.

**Tabelle 1**

| | Gew.-% |
|---|---|
| Decamethylcyclopentasiloxan | 42,9 |
| 2-Ethylhexylpalmitat | 5,0 |
| Triethylcitrat | 2,0 |
| Isopropylmyristat | 6,7 |
| Parfum | 6,7 |
| Bentone^{®} 38 V CG | 2,5 |
| Aluminiumchlorohydrat aP | 33,3 |
| Propylencarbonat | 0,5 |

In dem Aerosol-Konzentrat der Tabelle 1 wurde das folgende Handelsprodukt eingesetzt: Bentone^{®} 38 V CG - INCI-Bezeichnung: Disteardimonium Hectorite (Elementis Specialties)

### 2) Vergleich verschiedener Aerosole bei wechselnden Gewichtsverhältnissen der Ester 2-Ethylhexylpalmitat und Triethylcitrat

Wie in Ausführungsbeispiel 1 wurden acht Aerosol-Konzentrate der folgenden Zusammensetzungen (vgl. Tabelle 2 - Mengenangaben in [Gew.-%]) hergestellt. Diese wurden jeweils mit einem Propan/n-Butan-Gemisch (15/85) in einem Gewichtsverhältnis von 15 : 85 (Konzentrat: Treibgas) begast und in handelsübliche Aerosoldosen abgefüllt.

**Tabelle 2**

| | **1** | **2** | **3** | **4** | **5** | **6** | **7** | **8** |
|---|---|---|---|---|---|---|---|---|
| Decamethylcyclopentasiloxan | 42,9 | 42,9 | 42,9 | 42,9 | 42,9 | 42,9 | 42,9 | 42,9 |
| 2-Ethylhexylpalmitat | 7,0 | 6,0 | 5,0 | 4,0 | 3,0 | 2,0 | 1,0 | 0,0 |
| Triethylcitrat | 0,0 | 1,0 | 2,0 | 3,0 | 4,0 | 5,0 | 6,0 | 7,0 |
| Isopropylmyristat | 6,7 | 6,7 | 6,7 | 6,7 | 6,7 | 6,7 | 6,7 | 6,7 |
| Parfum | 6,7 | 6,7 | 6,7 | 6,7 | 6,7 | 6,7 | 6,7 | 6,7 |
| Bentone^{®} 38 V CG | 2,5 | 2,5 | 2,5 | 2,5 | 2,5 | 2,5 | 2,5 | 2,5 |
| Aluminiumchloro-hydrat aP | 33,3 | 33,3 | 33,3 | 33,3 | 33,3 | 33,3 | 33,3 | 33,3 |
| Propylencarbonat | 0,5 | 0,5 | 0,5 | 0,5 | 0,5 | 0,5 | 0,5 | 0,5 |

Anschließend wurden die Aerosole 1-8 im Abstand von jeweils 15 cm für jeweils 3 Sekunden auf einen Träger aus schwarzer Pappe gesprüht, wobei konstant auf einen Punkt auf der Pappe gesprüht wurde (d.h., dass die Aerosoldosen während des Sprühvorganges nicht bewegt wurden). Die ausgebrachten Mengen wurden durch Rückwiegen der Aerosoldosen bestimmt.
Die jeweils auf die Trägeroberfläche aufgebrachte Produktmenge wurde ebenfalls gravimetrisch bestimmt und der Flächeninhalt des jeweiligen, mit dem Produkt beladenen, runden Sprühflecks durch Messung des jeweiligen Radius ermittelt (Abbildung 1).

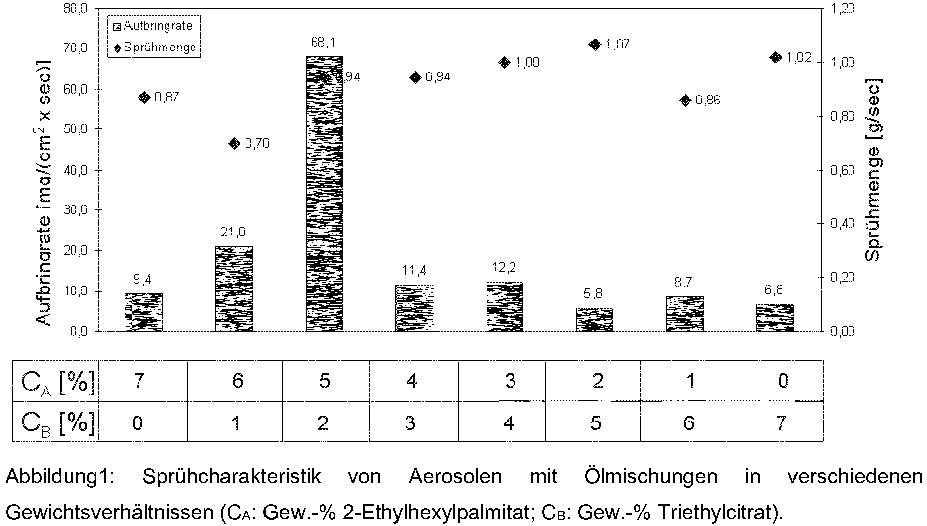

Abbildung 1 zeigt, dass die Aufbringrate bei dem erfindungsgemäßen Produkt überdurchschnittlich gut ist. Bereits bei einem Gewichtsverhältnis des 2-Ethylhexylpalmitats zum Triethylcitrat von 6 : 1 (Zusammensetzung des Beispiels 2) ist erkennbar, dass sich die Aufbringrate des Produktes deutlich gegenüber Produkten verbessert, die jeweils nur einen der beiden Ester enthalten (Zusammensetzungen der Beispiele 1 und 8). Werden die beiden zuvor genannten Ester jedoch annähernd im Gewichtsverhältnis von 1 : 1 eingesetzt, oder wird Triethylcitrat sogar im Überschuss zum 2-Ethylhexylpalmitat eingesetzt, so verschlechtert sich die Aufbringrate wieder (Zusammensetzungen der Beispiele 4-7).

## Patentansprüche

1. Aerosolzusammensetzung für die Körperpflege, enthaltend
- mindestens ein Treibmittel und
- mindestens eine Zusammensetzung A, enthaltend
a) mindestens einen Ester, der aus mindestens einem linearen oder verzweigten, gesättigten oder ungesättigten Alkohol mit 4 bis 30 Kohlenstoffatomen und mindestens einer linearen oder verzweigten, gesättigten oder ungesättigten Carbonsäure mit 8 bis 30 Kohlenstoffatomen gebildet wird, und
b) mindestens einen Ester, der aus mindestens einer C₂-C₇-Mono-, -Di- oder-Tricarbonsäure, die gegebenenfalls eine oder mehrere Hydroxylgruppen enthalten kann, und mindestens einem linearen oder verzweigten, gesättigten oder ungesättigten Alkohol mit 1 bis 30 Kohlenstoffatomen gebildet wird,
**dadurch gekennzeichnet,**
**dass** die Zusammensetzung A maximal 2 Gew.-% freies Wasser, bezogen auf das Gesamtgewicht der Zusammensetzung A, enthält, und
**dass** das Gewichtsverhältnis der Ester a) : b) in der Zusammensetzung A 3,5 : 1 bis 1,5 : 1 beträgt.

2. Desodorierende Aerosolzusammensetzung für die Körperpflege, enthaltend - bezogen auf ihr Gesamtgewicht -
- 10 bis 90 Gew.-% mindestens eines Treibmittels, ausgewählt aus Propan, n-Butan, Isobutan, n-Pentan, Isopentan, Dimethylether, Kohlendioxid, Distickstoffoxid, Fluorkohlenwasserstoffen und/oder Fluorchlorkohlenwasserstoffen, und
- 1 bis 40 Gew.-% mindestens eine Zusammensetzung A, enthaltend
a) mindestens einen Ester, der aus mindestens einem linearen oder verzweigten, gesättigten oder ungesättigten Alkohol mit 4 bis 30 Kohlenstoffatomen und mindestens einer linearen oder verzweigten, gesättigten oder ungesättigten Carbonsäure mit 8 bis 30 Kohlenstoffatomen gebildet wird,
b) mindestens einen Ester, der aus mindestens einer C₂-C₇-Mono-, -Di- oder-Tricarbonsäure, die gegebenenfalls eine oder mehrere Hydroxylgruppen enthalten kann, und mindestens einem linearen oder verzweigten, gesättigten oder ungesättigten Alkohol mit 1 bis 30 Kohlenstoffatomen gebildet wird, und
c) mindestens einen schweißhemmenden Wirkstoff,
**dadurch gekennzeichnet,**
**dass** die Zusammensetzung A maximal 2 Gew.-% freies Wasser, bezogen auf das Gesamtgewicht der Zusammensetzung A, enthält, und
**dass** das Gewichtsverhältnis der Ester a) : b) in der Zusammensetzung A 3,5 : 1 bis 1,5 : 1 beträgt.

3. Aerosolzusammensetzung nach Anspruch 1 oder desodorierende Aerosolzusammensetzung nach Anspruch 2, **dadurch gekennzeichnet, dass**
- der Ester a) aus mindestens einem verzweigten Alkohol mit 4 bis 20, bevorzugt mit 5 bis 18 und insbesondere mit 6 bis 16 Kohlenstoffatomen und mindestens einer linearen oder verzweigten, gesättigten oder ungesättigten Carbonsäure mit 8 bis 24, bevorzugt mit 10 bis 22 und insbesondere mit 12 bis 20 Kohlenstoffatomen gebildet wird, und
- der Ester b) aus mindestens einer C₂-C₇-Mono-, -Di- oder -Tricarbonsäure, die gegebenenfalls eine oder mehrere Hydroxylgruppen enthalten kann, und mindestens einem linearen oder verzweigten, gesättigten oder ungesättigten Alkohol mit 1 bis 10, bevorzugt mit 1 bis 7 und insbesondere mit 1 bis 4 Kohlenstoffatomen gebildet wird.

4. Aerosolzusammensetzung nach einem der Ansprüche 1 oder 3, oder desodorierende Aerosolzusammensetzung nach einem der Ansprüche 2 oder 3, **dadurch gekennzeichnet, dass**
- der Ester a) ausgewählt ist aus 2-Ethylhexyllaurat, 2-Ethylhexylmyristat, 2-Ethylhexylpalmitat, 2-Ethylhexylcocoat, 2-Ethylhexylstearat, 2-Ethylhexylisostearat, Hexyldecyllaurat, Hexyldecylstearat, Isooctylstearat, Isononylisononanoat, Isononylstearat, Isotridecylnonanoat, 2-Octyldodecylpalmitat, Isocetylstearat und/oder Mischungen dieser Ester, und
- der Ester b) ausgewählt ist aus den Methyl-, Ethyl, n-Propyl-, Isopropyl-, n-Butyl-, 2-Butyl- oder den tert.-Butylestern der Glycolsäure, Milchsäure, Äpfelsäure, Weinsäure, Zitronensäure, Oxalsäure, Malonsäure, Bernsteinsäure, Glutarsäure und/oder Adipinsäure.

5. Aerosolzusammensetzung nach einem der Ansprüche 1, 3 oder 4, oder desodorierende Aerosolzusammensetzung nach einem der Ansprüche 2, 3 oder 4, **dadurch gekennzeichnet, dass** sie als Ester a) 2-Ethylhexylpalmitat und der Ester b) Triethylcitrat enthält.

6. Aerosolzusammensetzung nach einem der Ansprüche 1, 3, 4 oder 5 oder desodorierende Aerosolzusammensetzung nach einem der Ansprüche 2, 3, 4 oder 5 **dadurch gekennzeichnet, dass** das Gewichtsverhältnis der Ester a) zu b) in der Zusammensetzung A 3 : 1 bis 2 : 1 beträgt.

7. Aerosolzusammensetzung nach einem der Ansprüche 1, 3, 4, 5 oder 6 oder desodorierende Aerosolzusammensetzung nach einem der Ansprüche 2, 3, 4, 5 oder 6, **dadurch gekennzeichnet, dass** sie
- 1 bis 30 Gew.-%, bevorzugt 2 bis 20 Gew.-%, mehr bevorzugt 3 bis 15 Gew.-% und besonders bevorzugt 5 bis 10 Gew.-% mindestens eines Esters a) und
- 1 bis 10 Gew.-%, bevorzugt 1 bis 7,5 Gew.-%, mehr bevorzugt 1 bis 5 Gew.-% und besonders bevorzugt 1 bis 3 Gew.-% mindestens eines Esters b) enthält,
wobei sich die Mengenangaben auf das Gesamtgewicht der Zusammensetzung A beziehen.

8. Aerosolzusammensetzung nach einem der Ansprüche 1, 3, 4, 5, 6 oder 7 oder desodorierende Aerosolzusammensetzung nach einem der Ansprüche 2, 3, 4, 5, 6 oder 7, **dadurch gekennzeichnet, dass** sie in der Zusammensetzung A zusätzlich mindestens ein weiteres, von a) und b) verschiedenes, unter Normalbedingungen flüssiges Öl als Träger enthält.

9. Aerosolzusammensetzung oder desodorierende Aerosolzusammensetzung nach Anspruch 8, **dadurch gekennzeichnet, dass** sie eine Mischung mindestens zwei von a) und b) verschiedenen, unter Normalbedingungen flüssigen Ölen, insbesondere bevorzugt eine Mischung aus mindestens zwei der folgenden Öle enthält: flüchtige cyclische Silikonöle wie Decamethylcyclopentasiloxan und/oder Dodecamethylcyclohexasiloxan, Esteröle wie Isopropylmyristat und/oder Isopropylpalmitat, und/oder Benzoesäureester von linearen oder verzweigten C₈-₂₂-Alkanolen.

10. Desodorierende Aerosolzusammensetzung nach einem der Ansprüche 2, 3, 4, 5, 6, 7, 8 oder 9, **dadurch gekennzeichnet, dass** sie als schweißhemmenden Wirkstoff mindestens einen schweißhemmenden Antitranspirant-Wirkstoff, bevorzugt ausgewählt aus adstringierenden Aluminiumsalzen, insbesondere Aluminiumchlorohydrat, Aluminiumsequichlorohydrat und/oder Aluminiumchlorid, in einer Gesamtmenge von 5 - 60 Gew.-%, vorzugsweise von 10 - 50 Gew.-% und insbesondere 15 - 40 Gew.-%, enthält, wobei sich die Mengenangaben auf das Gesamtgewicht der Zusammensetzung A beziehen.

## Claims

1. An aerosol composition for personal hygiene, containing
- at least one propellant and
- at least one composition A, which contains
a) at least one ester, which is formed of at least one linear or branched, saturated or unsaturated alcohol having from 4 to 30 carbon atoms and at least one linear or branched, saturated or unsaturated carboxylic acid having from 8 to 30 carbon atoms, and
b) at least one ester, which is formed of at least one C₂-C₇ monocarboxylic acid, dicarboxylic acid or tricarboxylic acid, which may contain one or more hydroxyl groups, and at least one linear or branched, saturated or unsaturated alcohol having from 1 to 30 carbon atoms,
**characterized in that**
composition A contains no more than 2 wt.% of free water, based on the total weight of composition A, and
**in that** the weight ratio of esters a) : b) in composition A is from 3.5 : 1 to 1.5 : 1.

2. A deodorizing aerosol composition for personal hygiene, containing
- from 10 to 90 wt.% of at least one propellant, selected from propane, n-butane, isobutane, n-pentane, isopentane, dimethyl ether, carbon dioxide, dinitrogen oxide, fluorocarbons and/or chlorofluorocarbons, and
- from 1 to 40 wt.% of at least one composition A, containing
a) at least one ester which is formed of at least one linear or branched, saturated or unsaturated alcohol having from 4 to 30 carbon atoms and at least one linear or branched, saturated or unsaturated carboxylic acid having from 8 to 30 carbon atoms,
b) at least one ester which is formed of at least one C₂-C₇ monocarboxylic acid, dicarboxylic acid or tricarboxylic acid, which may contain one or more hydroxyl groups, and at least one linear or branched, saturated or unsaturated alcohol having from 1 to 30 carbon atoms, and
c) at least one antiperspirant active ingredient,
based on the weight of said composition,
**characterized in that**
composition A contains no more than 2 wt.% of free water, based on the total weight of composition A, and
**in that** the weight ratio of esters a) : b) in composition A is from 3.5 : 1 to 1.5 : 1.

3. The aerosol composition according to claim 1 or the deodorizing aerosol composition according to claim 2, **characterized in that**
- ester a) is formed of at least one branched alcohol having from 4 to 20, preferably from 5 to 18 and in particular from 6 to 16 carbon atoms, and at least one linear or branched, saturated or unsaturated carboxylic acid having from 8 to 24, preferably from 10 to 22 and in particular from 12 to 20 carbon atoms, and
- ester b) is formed of at least one C₂-C₇ monocarboxylic acid, dicarboxylic acid or tricarboxylic acid, which may contain one or more hydroxyl groups, and at least one linear or branched, saturated or unsaturated alcohol having from 1 to 10, preferably from 1 to 7 and in particular from 1 to 4 carbon atoms.

4. The aerosol composition according to one of claims 1 or 3 or the deodorizing aerosol composition according to one of claims 2 or 3, **characterized in that**
- ester a) is selected from 2-ethylhexyl laurate, 2-ethylhexyl myristate, 2-ethylhexyl palmitate, 2-ethylhexyl cocoate, 2-ethylhexyl stearate, 2-ethylhexyl isostearate, hexyldecyl laurate, hexyldecyl stearate, isooctyl stearate, isononyl Isononanoate, isononyl stearate, isotridecyl nonanoate, 2-octyldodecyl palmitate, isocetyl stearate and/or mixtures of said esters, and
- ester b) is selected from methyl esters, ethyl esters, n-propyl esters, isopropyl esters, n-butyl esters, 2-butyl esters or tertiary butyl esters of glycolic acid, lactic acid, malic acid, tartaric acid, citric acid, oxalic acid, malonic acid, succinic acid, glutaric acid and/or adipic acid.

5. The aerosol composition according to one of claims 1, 3 or 4 or the deodorizing aerosol composition according to one of claims 2, 3 or 4, **characterized in that** said composition contains 2-ethylhexyl palmitate as ester a) and triethyl citrate as ester b).

6. The aerosol composition according to one of claims 1, 3, 4 or 5 or the deodorizing aerosol composition according to one of claims 2, 3, 4 or 5, **characterized in that** the weight ratio of ester a) to ester b) in composition A is fro 3 : 1 to 2 : 1.

7. The aerosol composition according to one of claims 1, 3, 4, 5 or 6 or the deodorizing aerosol composition according to one of claims 2, 3, 4, 5 or 6, **characterized in that** said composition contains
- from 1 to 30 wt.%, preferably from 2 to 20 wt.%, more preferably from 3 to 15 wt.% and particularly preferably from 5 to 10 wt.% of at least one ester a), and
- from 1 to 10 wt.%, preferably from 1 to 75 wt.%, more preferably from 1 to 5 wt.% and particularly preferably from 1 to 3 wt,% of at least one ester b),
the quantities stated relating to the total weight of composition A.

8. The aerosol composition according to one of claims 1, 3, 4, 5, 6 or 7 or the deodorizing aerosol composition according to one of claims 2, 3, 4, 5, 6 or 7, **characterized in that** said composition also contains at least one additional oil as the carrier in composition A, which oil is different from a) and b) and is liquid under normal conditions.

9. The aerosol composition or the deodorizing aerosol composition according to claim 8, **characterized in that** said composition contains a mixture of at least two oils, which are different from a) and b) and are liquid under normal conditions, particularly preferably a mixture of at least two of the following oils: volatile, cyclic silicone oils such as decamethylcyclopentasiloxane and/or dodecamethylcyclohexasiloxane, ester oils such as isopropyl myristate and/or isopropyl palmitate, and/or benzoic acid esters of linear or branched C₈₋₂₂ alkanols.

10. The deodorizing aerosol composition according to one of claims 2, 3, 4, 5, 6, 7, 8 or 9, **characterized in that** said composition contains, as the antiperspirant active ingredient, at least one antiperspirant active ingredient preferably selected from astringent aluminum salts, in particular aluminum chlorohydrate, aluminum sesquichlorohydrate and/or aluminum chloride, in a total amount of from 5 to 60 wt.%, preferably from 10 to 50 wt.% and in particular from 15 to 40 wt.%, the quantities stated relating to the total weight of composition A.

## Revendications

1. Composition d'aérosol pour les soins corporels contenant
- au moins un agent propulseur et
- au moins une composition A contenant
a) au moins un ester constitué d'au moins un alcool linéaire ou ramifié, saturé ou non-saturé, comportant 4 à 30 atomes de carbone et d'au moins un acide carboxylique linéaire ou ramifié, saturé ou non-saturé, comportant 8 à 30 atomes de carbone, et
b) au moins un ester, constitué d'au moins un acide mono-, di- ou tricarboxylique C₂-C₇, pouvant le cas échéant contenir un ou plusieurs groupes hydroxyles, et d'au moins un alcool linéaire ou ramifié, saturé ou non-saturé comportant 1 à 30 atomes de carbone,
**caractérisé en ce que**
la composition A contient au maximum 2 % en poids d'eau libre rapporté au poids total de la composition A, et
le rapport de poids des esters a) : b) dans la composition A s'élève de 3,5 : 1 à 1,5 : 1.

2. Composition d'aérosol désodorisante pour les soins corporels contenant, rapporté à son poids total -
- 10 à 90 % en poids d'au moins un agent propulseur, sélectionné parmi le propane, le n-butane, l'isobutane, le n-pentane, l'isopentane, lé diméthyléther, le dioxyde de carbone, le protoxyde d'azote, les hydrocarbures fluorés et/ou les chlorofluorocarbures, et
- 1 à 40 % en poids d'au moins une composition A, contenant
a) au moins un ester, constitué d'au moins un alcool linéaire ou ramifié, saturé ou non-saturé, comportant 4 à 30 atomes de carbone et d'au moins un acide carboxylique linéaire ou ramifié, saturé ou non-saturé, comportant 8 à 30 atomes de carbone,
b) au moins un ester, constitué d'au moins un acide mono-, di- ou tricarboxylique C₂-C₇, pouvant le cas échéant contenir un ou plusieurs groupes hydroxyles, et d'au moins un alcool linéaire ou ramifié, saturé ou non-saturé comportant 1 à 30 atomes de carbone, et
c) au moins un principe actif antisudoral,
**caractérisé en ce que**,
la composition A contient au maximum 2 % en poids d'eau libre, rapporté au poids total de la composition A, et
le rapport de poids des esters a) : b) dans la composition A s'élève de 3,5 : 1 à 1,5 : 1.

3. Composition d'aérosol selon la revendication 1 ou composition d'aérosol désodorisante selon la revendication 2, **caractérisée en ce que**
- l'ester a) est constitué d'au moins un alcool ramifié comportant 4 à 20, préférablement 5 à 18 et notamment 6 à 16 atomes de carbone et d'au moins un acide carboxylique linéaire ou ramifié, saturé ou non-saturé, comportant 8 à 24, préférablement 10 à 22 et notamment 12 à 20 atomes de carbone, et
- l'ester b) est constitué d'au moins un acide mono-, di- ou tricarboxylique C₂-C₇, pouvant le cas échéant contenir un ou plusieurs groupes hydroxyles, et d'au moins un alcool linéaire ou ramifié, saturé ou non-saturé comportant 1 à 10, préférablement 1 à 7 et notamment 1 à 4 atomes de carbone.

4. Composition d'aérosol selon l'une quelconque des revendications 1 ou 3, ou composition d'aérosol désodorisante selon l'une quelconque des revendications 2 ou 3, **caractérisée en ce que**
- l'ester a) est sélectionné parmi le laurate de 2-éthylhexyle, le myristate de 2-éthylhexyle, le palmitate de 2-ethylhexyle, le cocoate de 2-éthylhexyle, le stéarate de 2-éthylhexyle, l'isostéarate de 2-éthylhexyle, le laurate d'hexyldécyle, le stéarate d'hexyldécyle, le stéarate d'isooctyle, l'isononanoate d'isononyle, le stéarate d'isononyle, le nonanoate d'isotridécyle, le palmitate de 2-octyldodécyle, le stéarate d'isocétyle et/ou des composés de ces esters, et
- l'ester b) est sélectionné parmi les esters de méthyle, d'éthyle, de n-propyle, d'isopropyle, de n-butyle, de 2-butyle ou de butyle tertiaire de l'acide glycolique, de l'acide lactique, de l'acide malique, de l'acide tartrique, de l'acide citrique, de l'acide oxalique, de l'acide malonique, de l'acide succinique, de l'acide glutarique et/ou de l'acide adipique.

5. Composition d'aérosol selon l'une quelconque des revendications 1, 3 ou 4 ou composition d'aérosol désodorisante selon l'une quelconque des revendications 2, 3 ou 4, **caractérisée en ce qu'**elle contient du palmitate de 2-éthylhexyl en tant qu'ester a) et du citrate de triéthyle en tant qu'ester b).

6. Composition d'aérosol selon l'une quelconque des revendications 1, 3, 4 ou 5 ou composition d'aérosol selon l'une quelconque des revendications 2, 3, 4 ou 5 **caractérisée en ce que** le rapport de poids des esters a) sur b) dans la composition A s'élève de 3 : 1 à 2 : 1.

7. Composition d'aérosol selon l'une quelconque des revendications 1, 3, 4, 5 ou 6 ou composition d'aérosol désodorisante selon l'une quelconque des revendications 2, 3, 4, 5 ou 6, **caractérisée en ce qu'**elle contient
- 1 à 30 % en poids, préférablement 2 à 20 % en poids, plus préférablement 3 à 15 % en poids et très préférablement 5 à 10 % en poids d'au moins un ester a) et
- 1 à 10 % en poids, préférablement 1 à 7,5 % en poids, plus préférablement 1 à 5 % en poids et très préférablement 1 à 3 % en poids d'au moins un ester b),
dans laquelle les indications de quantité se rapportent au poids total de la composition A.

8. Composition d'aérosol selon l'une quelconque des revendications 1, 3, 4, 5, 6 ou 7 ou composition d'aérosol désodorisante selon l'une quelconque des revendications 2, 3,4, 5, 6 ou 7, **caractérisée en ce qu'**elle contient en plus dans la composition A au moins une autre huile liquide en conditions normales différente de a) et de b), en tant que support.

9. Composition d'aérosol ou composition d'aérosol désodorisante selon la revendication 8, **caractérisée en ce qu'**elle contient un composé d'au moins deux huiles liquides en conditions normales différentes de a) et de b), notamment préférablement un composé d'au moins deux des huiles suivantes ; des huiles de silicone cycliques volatiles comme le décaméthylcyclopentasiloxane et/ou le dodécaméthylcyclohexasiloxane, des huiles d'ester comme le myristate d'isopropyle et/ou le palmitate d'isopropyle, et/ou l'ester d'acide benzoïque d'alcanols C₆-₂₂ linéaires ou ramifiés.

10. Composition d'aérosol désodorisante selon l'une quelconque des revendications 2, 3, 4, 5, 6, 7, 8 ou 9, **caractérisée en ce qu'**elle contient en tant que principe actif antisudoral au moins un principe actif antitranspirant antisudoral, préférablement sélectionné parmi les sels d'aluminium astringents, notamment le chlorhydrate d'aluminium, le sequichlorhydrate d'aluminium et/ou le chlorure d'aluminium, dans une quantité totale de 5 à 60 % en poids, préférablement de 10 à 50 % en poids et notamment de 15 à 40 °C en poids, dans laquelle les indications de quantité se rapportent au poids total de la composition A.
